# EUROPÄISCHE PATENTSCHRIFT

(11) **EP 0 825 865 B1**
(45) Veröffentlichungstag und Bekanntmachung des Hinweises auf die Patenterteilung: **07.11.2001**
(21) Anmeldenummer: 96919765.6
(22) Anmeldetag: 10.05.1996
(51) Int. Cl.: A61K 31/565, A61K 9/70

(54) **TRANSDERMALES THERAPEUTISCHES SYSTEM (TTS) ZUR VERABREICHUNG VON TESTOSTERON**
TRANSDERMAL THERAPEUTIC SYSTEM FOR ADMINISTERING TESTOSTERONE
SYSTEME THERAPEUTIQUE TRANSDERMIQUE POUR ADMINISTRER DE LA TESTORERONE

(30) Priorität: 10.05.1995 DE 19517145
(43) Veröffentlichungstag der Anmeldung: 04.03.1998
(73) Patentinhaber: HEXAL AG, D-83607 Holzkirchen (DE)
(72) Erfinder: FISCHER, Wilfried, D-83607 Holzkirchen (DE); BRACHER, Daniel, D-83607 Holzkirchen (DE)
(74) Vertreter: Boeters, Hans Dietrich, Dr.
(86) Internationale Anmeldenummer: EP9602013
(87) Internationale Veröffentlichungsnummer: WO9635427

(56) Entgegenhaltungen:
- EP-A- 0 563 507
- WO-A-89/07959
- WO-A-92/10231
- WO-A-92/20377
- WO-A-93/00058
- WO-A-93/25168
- WO-A-94/23707
- GB-A- 2 161 073
- GB-A- 2 185 187
- CHEMICAL ABSTRACTS, vol. 115, no. 12, 23.September 1991 Columbus, Ohio, US; abstract no. 119907, XP002013566 & EUR. J. PHARM. BIOPHARM., Bd. 37, Nr. 1, 1991, Seiten 30-33, T. LOFTSSON ET AL.: "THE EFFECTS OF CYCLODEXTRINS ON TRANSDERMAL DELIVERY OF DRUGS"

## Beschreibung

Testosteron und Testosteron-Derivate werden nach dem heutigen Stand der Wissenschaft vornehmlich für die Hormonsubstitutionstherapie bei Männern eingesetzt. Weitere Indikationsstellungen neben primärem und sekundärem Hypogonadismus sind Oligozoospermie, Impotentia coeundi und Ejaculatio preacox. Ferner sind Untersuchungen über die Anwendung bei der Osteoporose des Mannes im Gang; vergleiche beispielsweise Rapado et al. in Trends in Osteoporosis, 1 (1989) 1-9.

Infolge des hohen First-pass-Effekts ist Testosteron bei oraler Gabe weitgehend unwirksam. Daher werden in der Substitutionstherapie entweder oral wirksame Ester, wie Testosteron-undecanoat, oder injizierbare Depotpräparate, wie Testosteron-enantat und Testosteron-propionat, verabreicht.

Aus diesen Daten ergeben sich mehrere Ansatzpunkte für eine sinnvolle Entwicklung von transdermalen therapeutischen Systemen (TTS). Mit derartigen Systemen lassen sich die bei oraler Anwendung hohen Wirkstoffdosen vermeiden, läßt sich eine schmerzhafte Injektion umgehen, der Blutspiegel besser steuern und besser an den circadianen Rhythmus anpassen.

So beschreibt US-A-4 704 282 ein transdermales therapeutisches System mit einer Trägerfolie (reenforcing means), einem Wirkstoffreservoir und einer entfernbaren Abdeckfolie (release liner). Das Wirkstoffreservoir kann in Form eines wässerigen oder nicht-wässerigen Gels oder Polymermaterials vorliegen, wobei der Wirkstoff darin in einer Konzentration gelöst vorliegt, die nicht größer als die Sättigungskonzentration des Wirkstoffs im Matrixmaterial ist. Die Matrix kann Permeationsenhancer enthalten. Bei dem zu verabreichenden Wirkstoff kann es sich beispielsweise um Testosteron handeln. Liegt allerdings bei einem transdermalen therapeutischen System Testosteron in untersättigter Lösung vor, so ist für befriedigende Blutspiegel die Gegenwart von Permeationsenhancern unverzichtbar; vergleiche WO-A-9 210 231, Seite 11, Zeile 33.

Mit US-A-4 849 224 wird ein transdermales therapeutisches System vorgeschlagen, bei dem ein Wirkstoffreservoir von einer Trägerfolie (backing foil) und einer porösen Membran gebildet wird. Bei dem Wirkstoff kann es sich um Testosteron oder einen Testosteron-ester handeln. Neben dem Wirkstoff können Permeationsenhancer vorgesehen sein. Die Brauchbarkeit dieses bekannten Systems ist allerdings insofern kritisch zu sehen, als das Wirkstoffreservoir, das durch das Laminat aus Trägerfolie und Membran gebildet wird, mit Hilfe eines Klebemittelringes auf der Haut haften soll. Indem das Klebemittel ringförmig ausgebildet wird, wird praktisch ein Fenster vorgesehen, durch das der Wirkstoff mit dem Permeationsenhancer über die Membran zur Haut vordringen kann, ohne daß der Permeationsenhancer unnötig mit dem Klebemittel in Berührung kommt, wobei zusätzlich zum Schutz des Klebemittels noch ringförmige Dichtungselemente vorgesehen sind.

Da mit einer ringförmigen Klebemittelzone nur eine begrenzte Haftung erreicht werden kann, ist die Größe des bekannten Pflasters beschränkt.

WO-A-9 210 231 = EP-A-0 562 041 greift den Stand der Technik von US-A-4 849 224 auf, wonach für die Haftung eines transdermalen therapeutischen Systems an der Haut ein Klebemittelring vorgesehen wird; vergleiche WO-A-9 210 231, Seite 13, Zeilen 18/19. Alternativ werden jedoch eine basale Klebemittelschicht, mit der das Reservoir unterlegt ist (separate basal adhesive layer underlaying the reservoir), und ferner ein Klebemittelüberzug für das Reservoir (adhesive overlay for the reservoir) vorgeschlagen. Da nach diesem Stand der Technik jedoch der Einsatz eines Permeationsenhancers bei der Applikation von Testosteron unverzichtbar ist, ist nicht zu erkennen, wie WO-A-9 210 231 das unerwünschte Eindringen des Permeationsenhancers in das Klebemittel verhindern will, wenn das Klebemittel nicht ringförmig vorgesehen wird.

Ferner beschreiben WO-A-9 503 764, WO-A-9 423 707 und US-A-5 152 997 transdermale therapeutische Systeme zur Verabreichung von Testosteron, wobei es sich bei den Systemen um Reservoir-Systeme handelt, bei denen der Wirkstoff in ungesättigter Form vorliegt und ein Permeationsbeschleuniger vorgesehen ist. Diese bekannten Lehren entsprechen daher dem Stand der Technik, wie er durch WO-A-9 210 231 Seite 11 Zeile 33 diskutiert wird.

Der Erfindung liegt nun die Aufgabe zugrunde, ein transdermales therapeutisches System zur Verabreichung von Testosteron oder einem Testosteron-Derivat vorzusehen, das auf den Einsatz von Permeationsenhancern verzichtet und befriedigende Wirkstoffspiegel im Blut garantiert.

Dazu wird erfindungsgemäß ein transdermales therapeutisches System (TTS) zur Verabreichung von Testosteron oder einem Testosteron-Derivat als Wirkstoff mit
- einer Trägerfolie (backing foil),
- einem Reservoir,
- einem alkoholischen Wirkstoffträger,
- einer Klebemittelschicht für den Hautkontakt des Systems und
- einer entfernbaren Abdeckfolie (release liner) vorgeschlagen, wobei
- das Systen außer dem alkoholischen Wirkstoffträger keinen Permeationsenhancer umfaßt,
- der Wirkstoff in dem Wirkstoffträger gesättigt vorliegt und
- zwischen dem Reservoir und der Klebemittelschicht eine Membran vorgesehen ist,
- die die Wirkstoffreigabe nicht steuert, während die Klebemittelschicht die Wirkstoffreigabe steuert.

Der Erfindung liegt die überraschende Beobachtung zugrunde, daß sich ein befriedigender Wirkstoffspiegel im Blut auch bzw. gerade dann erreichen läßt, wenn der Wirkstoff in dem Wirkstoffträger gesättigt vorliegt, obgleich WO-A-9 210 231 eine Untersättigung für den Wirkstoff fordert.

Erfindungsgemäß kann man einen Testosteron-ester, insbesondere Testosteron-enantat, Testosteron-cipionat, Testosteron-propionat oder Testosteron-undecanoat, oder ein Niederalkyl-testosteron, insbesondere Methyl-testosteron, als Testosteron-Derivat verwenden.

Der Wirkstoff kann dabei als Komplex mit Cyclodextrin oder Cyclodextrin-Derivaten vorliegen, insbesondere mit ß-Cyclodextrin.

Bei der Membran kann es sich um eine Polyethylen- oder eine Polypropylenmembran handeln.

Gemäß einer bevorzugten Ausführungsform wird das Reservoir durch die Trägerfolie und die Membran gebildet.

Bei dem alkoholischen Wirkstoffträger kann es sich erfindungsgemäß um Ethanol oder einen niedermolekularen einwertigen Alkohol, wie Isopropanol, oder einen niedermolekularen mehrwertigen Alkohol handeln, beispielsweise Propylenglycol, oder um deren Gemische.

Für die Klebemittelschicht kann man erfindungsgemäß ein druckempfindliches alkoholresistentes Klebemittel beispielsweise auf Polyurethanbasis, Isobutylenbasis, Polyvinyletherbasis, Siliconbasis oder Acrylatbasis wählen.

Bei dem Klebemittel auf Siliconbasis kann es sich um Silicon-Kleber handeln, welche auf zwei Hauptbestandteilen basieren: Ein Polymer oder Klebstoff, insbesondere Polysiloxan, und ein tackerhöhendes Harz. Der Polysiloxankleber ist gewöhnlich mit einem Vernetzer für den Kleber, typischerweise einem hochmolekularen Polydiorganosiloxan, und mit dem Harz zubereitet, um über ein angemessenes organisches Lösungsmittel eine dreidimensionale Silicatstruktur zu ergeben. Die Zumischung des Harzes zu Polymer ist der wichtigste Faktor, um die physikalischen Eigenschaften der polysiloxanen Kleber zu ändern; vgl. beispielsweise Sobieski, et al., "Silicone Pressure Sensitive Adhesives", Handbook of Pressure Sensitive Adhesive Technology, 2nd ed., pp. 508-517 (D. Satas, ed.), Van Nostrand Reinhold, New York (1989).

Passende druckempfindliche Silikonkleber sind im Handel unter dem Warenzeichen BIO-PSA X7 erhältlich.

Ein weiteres Beispiel für ein druckempfindliches Klebemittel auf Siliconbasis ist trimethyliertes Siliciumdioxid, das mit Polydimethylsiloxan mit endständigen Trimethylsiloxy-Gruppen behandelt worden ist.

Bei dem Klebemittel auf Acrylatbasis kann es sich um ein beliebiges Homopolymer, Copolymer oder Terpolymer, bestehend aus verschiedenen Acrylsäurederivaten handeln.

So können die Acrylatpolymere Polymere eines oder mehrerer Monomere von Acrylsäuren und anderen copolymerisierbaren Monomeren sein. Außerdem können die Acrylatpolymere Copolymere von Alkylacrylaten und/oder -methacrylaten und/oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 40 % eines anderen Monomeren.

Im folgenden sind Acrylatmonomere aufgeführt, die mit Acrylsäure, Methacrylsäure, Butylacrylat, Butylmetharylat, Hexylacrylat, Hexylmethacrylat, Isooctylacrylat, Isooctylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat verwendet werden können.

So können funktionelle Monomere, die mit den oben genannten Acrylaten, Methacrylaten, Alkylacrylaten oder -methacrylaten copolymerisierbar sind, eingesetzt werden, beispielsweise Acrylsäure, Methacrylsäure, Maleinsäure, Maleinanhydrid, Hydroxyethylacrylat, Hydroxypropylacrylat, Acrylamid, Dimethylacrylamid, Acrylnitril, Dimethylaminoethylacrylat, Dimethylaminoethylmethacrylat, tert-Butylaminoethylacrylat, tert-Butylaminoethylmethacrylat, Methoxyethylacrylat und Methoxyethylmethacrylat.

Weitere Einzelheiten und Beispiele für druckempfindliche Acrylate, welche für die Erfindung geeignet sind, sind in Satas Handbook of Pressure Sensitive Adhesive Technology "Acrylic Adhesives", 2nd ed., pp. 396-456 (D. Satas, ed.), Van Nostrand Reinhold, New York (1989) beschrieben.

Passende druckempfindliche Acrylate sind im Handel erhältlich.

Das erfindungsgemäße transdermale therapeutische System kann zusätzlich α-Tocopherol oder ein α-Tocopherolderivat und/oder ein viskositätserhöhendes Mittel, wie Hydroxypropylcellulose, enthalten.

Nachstehend wird die Erfindung durch eine Figur und ein Beispiel näher erläutert.

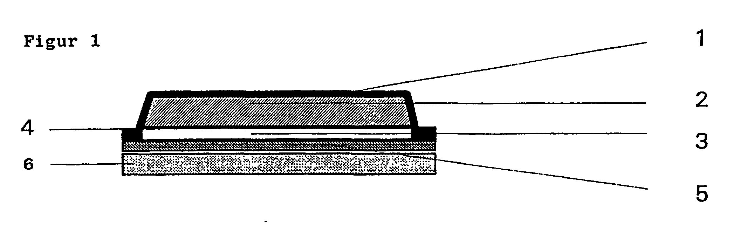

Nach **Figur 1** kann ein erfindungsgemäßes transdermales therapeutisches System eine Trägerfolie (backing foil) (1) aufweisen, die leicht konvex ausgebildet sein kann, die mit einer Membran (3) ein Reservoir (2) bildet, wobei die Membran (3) die Wirkstoffreigabe nicht steuert. Auf der Membran (3) ist nach **Figur 1** eine Klebemittelschicht (5) aufgebracht, die die Wirkstoffreigabe steuert. Diese Klebemittelschicht (5) ist wiederum mit einer Abdeckfolie (release liner) (6) abgedeckt, die vor der Applikation abzuziehen ist. Nach der Figur kann die Trägerfolie (1) mit einem ringförmigen Wulst (4) versehen sein, der auf dem umlaufenden Randbereich der Klebemittelschicht (5) aufliegt und im Auflagebereich mit der Membran (3) versiegelt ist.

### Beispiel

Für die Klebemittelschicht wurde ein druckempfindliches Klebemittel auf Siliconbasis verwendet (trimethyliertes Siliciumdioxid, das mit Polydimethylsiloxan mit endständigen Trimethylsiloxy-Gruppen behandelt worden ist; Schichtdicke trocken (SD) etwa 35 bis 45 *µ*m; Flächengewicht (FG) etwa 50 bis 60 g/m²). Eine Abdeckfolie aus PET (Dicke etwa 75 *µ*m; FG etwa 100 g/m²) wurde mit dem Silicon-Kleber mit Hilfe einer Beschichtungsanlage beschichtet. Auf die beschichtete Abdeckfolie wurde eine microporöse Polyethylenmembran oder eine microporöse Polypropylenmembran (heißsiegelfähig; Dicke etwa 50 *µ*m; FG etwa 10 g/m²) aufkaschiert, so daß ein Laminat aus Abdeckfolie, Klebemittel und Membran hergestellt wurde. Danach wurde das Laminat mit Hilfe einer Siegelmaschine (mit Schweißring) mit einer Trägerfolie aus Polyester (aluminiumbedampft mit Polyolefin-Siegelschicht (heißsiegelfähig); Dicke etwa 70 *µ*m) derart verschweißt, daß ein Spalt zum Einfüllen einer Wirkstofflösung zurückblieb. Das befüllbare transdermale therapeutische System (Leer-TTS) wurde beispielsweise mit einer Hamilton-Spritze oder mit einer Schlauchpumpe mit Kanüle mit der folgenden Wirkstofflösung befüllt:

Zusammensetzung der Wirkstofflösung pro TTS:

| | mg/TTS |
|---|---|
| Testosteron | 60,56 |
| 96-proz. Ethanol | 157,18 |
| Propylenglycol | 48,11 |
| α-Tocopherol | 56,78 |
| Hydroxypropylcellulose | 5,98 |
| Insgesamt | 328,61 |
| Füllvolumen des Reservoirs | 360 *µ*l |
| Dichte der Wirkstofflösung | 0,91285 g/cm³ |
| Füllmenge des Reservoirs | 328,6 mg |

Nach dem Befüllen wurde der Befüllungsspalt verschweißt. Befüllte transdermale therapeutische Systeme wurden mit Hilfe einer Stanze ausgestanzt.

## Patentansprüche

1. Transdermales therapeutisches System (TTS) zur Verabreichung von Testosteron oder einem Testosteron-Derivat als Wirkstoff mit
- einer Trägerfolie (backing foil),
- einem Reservoir,
- einem alkoholischen Wirkstoffträger,
- einer Klebemittelschicht für den Hautkontakt des Systems und
- einer entfernbaren Abdeckfolie (release liner), wobei
- das System außer dem alkoholischen Wirkstoffträger keinen Permeationsenhancer umfaßt,
- der Wirkstoff in dem Wirkstoffträger gesättigt vorliegt und
- zwischen dem Reservoir und der Klebemittelschicht eine Membran vorgesehen ist, die die Wirkstoffreigabe nicht steuert, während die Klebemittelschicht die Wirkstoffreigabe steuert.

2. System nach Anspruch 1, **gekennzeichnet durch** einen Testosteron-ester, insbesondere Testosteron-enantat, Testosteron-cipionat, Testosteron-propionat oder Testosteron-undecanoat, oder ein Niederalkyl-testosteron, insbesondere Methyl-testosteron, als Testosteron-Derivat.

3. System nach Anspruch 1 oder 2, **dadurch gekennzeichnet, daß** der Wirkstoff als Komplex mit Cyclodextrin oder einem Cyclodextrin-Derivat, insbesondere β-Cyclodextrin, vorliegt.

4. System nach einem der vorhergehenden Ansprüche, **dadurch gekennzeichnet, daß** das Reservoir durch die Trägerfolie und die Membran gebildet wird.

5. System nach einem der vorhergehenden Ansprüche, **gekennzeichnet durch** Ethanol oder einen niedermolekularen einwertigen oder mehrwertigen Alkohol, insbesondere Propylenglycol, oder deren Gemische als alkoholischer Wirkstoffträger.

6. System nach einem der vorhergehenden Ansprüche, **gekennzeichnet durch** eine Klebemittelschicht aus einem druckempfindlichen alkoholresistenten Klebemittel auf Polyurethanbasis, Isobutylenbasis, Polyvinyletherbasis, Siliconbasis oder Acrylatbasis.

7. System nach einem der vorhergehenden Ansprüche, **dadurch gekennzeichnet, daß** der Wirkstoffträger zusätzlich α-Tocopherol oder ein α-Tocopherol-Derivat enthält.

8. System nach einem der vorhergehenden Ansprüche, **dadurch gekennzeichnet, daß** der Wirkstoffträger zusätzlich ein viskositätserhöhendes Mittel, wie Hydroxypropylcellulose, enthält.

## Claims

1. Transdermal therapeutic system (TTS) for the administration of testosterone or a testosterone derivative as active ingredient, having
- a carrier film (backing foil),
- a reservoir,
- an alcoholic carrier for the active ingredient,
- an adhesive layer for contact of the system with the skin, and
- a removable cover film (release liner),
in which
- the system does not comprise a permeation enhancer with the exception of the alcoholic carrier for the active ingredient,
- the active ingredient is present in saturation in the carrier for the active ingredient, and
- there is provided between the reservoir and the adhesive layer a membrane which does not control the release of active ingredient, whilst the adhesive layer does control the release of active ingredient.

2. System according to claim 1, **characterised by** a testosterone ester, especially testosterone enanthate, testosterone cipionate, testosterone propionate or testosterone undecanoate, or a lower alkyl testosterone, especially methyl testosterone, as testosterone derivative.

3. System according to claim 1 or claim 2, **characterised in that** the active ingredient is present in the form of a complex with cyclodextrin or a cyclodextrin derivative, especially β-cyclodextrin.

4. System according to any one of the preceding claims, **characterised in that** the reservoir is formed by the carrier film and the membrane.

5. System according to any one of the preceding claims, **characterised by** ethanol or a low molecular weight monohydric or polyhydric alcohol, especially propylene glycol, or mixtures thereof, as alcoholic carrier for the active ingredient.

6. System according to any one of the preceding claims, **characterised by** an adhesive layer of a pressure-sensitive, alcohol-resistant adhesive based on polyurethane, isobutylene, polyvinyl ether, silicone or acrylate.

7. System according to any one of the preceding claims, **characterised in that** the carrier for the active ingredient additionally comprises α-tocopherol or an α-tocopherol derivative.

8. System according to any one of the preceding claims, **characterised in that** the carrier for the active ingredient additionally comprises a viscosity-increasing agent, such as hydroxypropyl cellulose.

## Revendications

1. Système thérapeutique transdermique (TTS) pour l'administration de testostérone, ou d'un dérivé de testostérone à titre de principe actif, comprenant :
- un film de support (backing foil),
- un réservoir,
- un support alcoolique de principe actif,
- une couche de produit adhésif pour le contact du système sur la peau, et
- un film de couverture à enlever (release liner),
dans lequel :
- le système ne comprend aucun produit de renfort de perméation autre que le support alcoolique de principe actif,
- le principe actif est présent sous forme saturée dans le support de principe actif, et
- il est prévu entre le réservoir et la couche d'adhésif une membrane qui ne commande par la libération du principe actif, tandis que la couche d'adhésif commande la libération du principe actif.

2. Système selon la revendication 1, **caractérisé par** un ester de testostérone, en particulier énantate de testostérone, cipionate de testostérone, propionate de testostérone, ou undécanoate de testostérone, ou testostérone à faible taux d'alkyle, en particulier méthyle-testostérone, à titre de dérivé de testostérone.

3. Système selon l'une ou l'autre des revendications 1 et 2, **caractérisé en ce que** le principe actif est présent sous forme complexe avec de la cyclodextrine, ou un dérivé de cyclodextrine, en particulier □-cyclodextrine.

4. Système selon l'une des revendications précédentes, **caractérisé en ce que** le réservoir est formé par le film de support et par la membrane.

5. Système selon l'une des revendications précédentes, **caractérisé par** de l'éthanol, ou par un alcool à faible poids moléculaire, mono-alcool ou poly-alcool, en particulier propylène glycol, ou leurs mélanges, à titre de support alcoolique pour le principe actif.

6. Système selon l'une des revendications précédentes, **caractérisé par** une couche d'adhésif formé d'un adhésif résistant à l'alcool et sensible à la pression, à base de polyuréthane, à base d'isobutyle, à base de polyvinyl-éther, à base de silicone, ou à base d'acrylate.

7. Système selon l'une des revendications précédentes, **caractérisé en ce que** le support de principe actif contient en supplément du □-tocophérol, ou un dérivé de □-tocophérol.

8. Système selon l'une des revendications précédentes, **caractérisé en ce que** le support de principe actif contient en supplément un produit augmentant la viscosité, comme de la hydroxypropylcellulose.
